(19) Europäisches Patentamt / European Patent Office / Office européen des brevets

(11) **EP 3 768 228 B1**

(12) **FASCICULE DE BREVET EUROPEEN**

(45) Date de publication et mention de la délivrance du brevet:
**14.08.2024 Bulletin 2024/33**

(21) Numéro de dépôt: **19709978.1**

(22) Date de dépôt: **18.03.2019**

(51) Classification Internationale des Brevets (IPC):
**A61K 8/34** (2006.01)    **A61K 8/35** (2006.01)
**A61K 8/37** (2006.01)    **A61Q 1/02** (2006.01)

(52) Classification Coopérative des Brevets (CPC):
**A61K 8/35; A61K 8/347; A61K 8/37; A61Q 1/02;**
A61K 2800/52

(86) Numéro de dépôt international:
**PCT/EP2019/056669**

(87) Numéro de publication internationale:
**WO 2019/179921 (26.09.2019 Gazette 2019/39)**

(54) **COMPOSITION UTILE POUR RALENTIR LA DÉGRADATION CAUSÉE PAR LA LUMIÈRE, LA CHALEUR ET/OU L'OXYDATION D'UN COMPOSÉ COSMÉTIQUEMENT ACTIF**

ZUSAMMENSETZUNG ZUR VERLANGSAMUNG DES ABBAUS DURCH LICHT, WÄRME UND/ODER OXIDATION EINER KOSMETISCH AKTIVEN VERBINDUNG

COMPOSITION USEFUL FOR SLOWING DOWN DEGRADATION CAUSED BY LIGHT, HEAT AND/OR OXIDATION OF A COSMETICALLY ACTIVE COMPOUND

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **19.03.2018 FR 1852336**

(43) Date de publication de la demande:
**27.01.2021 Bulletin 2021/04**

(73) Titulaire: **Sensient Cosmetic Technologies 95310 Saint Ouen L'Aumone (FR)**

(72) Inventeurs:
• **GUYOT-FERRÉOL, Véronique 92250 LA GARENNE COLOMBES (FR)**
• **CHAIGNE, Solène 95300 PONTOISE (FR)**

(74) Mandataire: **Lavoix 2, place d'Estienne d'Orves 75441 Paris Cedex 09 (FR)**

(56) Documents cités:
**EP-A1- 2 923 690    FR-A1- 2 871 686**

• **DATABASE GNPD [online] MINTEL; 28 June 2022 (2022-06-28), ANONYMOUS: "Shampoo", XP093128029, retrieved from https://www.gnpd.com/sinatra/recordpage/9693 118/ Database accession no. 9693118**

Remarques:
Le dossier contient des informations techniques présentées postérieurement au dépôt de la demande et ne figurant pas dans le présent fascicule.

## Description

La présente invention a trait à des compositions permettant de stabiliser des formulations, notamment des formulations cosmétiques, dermo-cosmétiques et/ou pharmaceutiques, contre les phénomènes de dégradation dues à la lumière, à la chaleur et/ou à l'oxydation.

Dans les domaines de la cosmétique, de la dermo-cosmétique et de la pharmacie, de nombreuses formulations comprennent un ou plusieurs composé(s) actif(s) choisi(s) parmi les colorants, les parfums, les antioxydants et les vitamines. Ce composé actif, d'origine naturelle ou synthétique, est notamment destiné à améliorer l'aspect des formulations, leur conférer une odeur caractéristique ou leur donner des propriétés spécifiques.

Souvent, de telles formulations sont conditionnées dans des emballages transparents à la lumière, par exemple en verre ou en matière plastique, de façon à les laisser apparaître. Ces emballages sont généralement également transparents aux rayons ultraviolets (UV).

De plus, les formulations peuvent être soumises à des variations de températures au cours de leur transport et/ou de leur stockage.

Or, certains composés actifs sont sensibles à la lumière, plus particulièrement aux rayons ultraviolets particulièrement énergétiques, et/ou à la chaleur. Ils sont alors susceptibles de se dégrader, ce qui peut avoir un impact négatif sur l'aspect et/ou les propriétés des formulations.

Par exemple, dans le cas des colorants, l'exposition à des rayons ultraviolets peut induire des réactions radicalaires modifiant la structure chimique des colorants et de ce fait leurs propriétés colorantes. On observe alors une modification de la couleur des formulations, sous forme de décoloration et/ou d'un virement de couleur.

Pour améliorer la stabilité des formulations colorées vis-à-vis des rayons ultraviolets, on peut utiliser des filtres ultraviolets, qui assurent une protection par absorption sélective du rayonnement UV.

A titre de filtres ultraviolets, on utilise notamment des composés de la famille des benzophénones ou d'autres composés absorbant les rayons ultraviolets, tels que ceux mentionnés par exemple dans l'annexe VII de la Directive C.E.E. N° 76/768 (relative aux filtres ultraviolets acceptés en cosmétique) ou dans les Directives de la F.D.A. (Food and Drugs Administration américaine), à savoir dans le Federal Register, vol. 43, n° 166 (25 août 1978).

Ces composés sont toutefois destinés à protéger la peau et les cheveux contre les effets nocifs des rayons UV, et non pas les formulations elles-mêmes ou leurs composés actifs, comme les colorants. A ce sujet, on pourra notamment se reporter à l'article "Filtres et écrans solaires" de M.C. Poelman dans « Actifs et additifs en cosmétologie », 2ème édition, 1999, Editions Tec & Doc, Paris.

L'utilisation d'un seul filtre UV dans une formulation n'assure toutefois pas toujours une protection suffisamment efficace de la coloration vis-à-vis des rayons ultraviolets. Le brevet EP 1 755 543 B1 décrit ainsi l'utilisation d'une composition stabilisante à titre d'additif stabilisateur de la coloration d'une formulation et consistant en une association de trois filtres ultra-violets appartenant respectivement à la famille des cinnamates, à celle des dibenzoylméthanes et à celle des salicylates. Cependant, le 2-éthylhexyl-méthoxycinnamate est suspecté d'être un perturbateur endocrinien par les instances règlementaires européennes. De plus, ce brevet est silencieux sur la capacité de la formulation à protéger d'autres actifs cosmétiques que les colorants.

D'autres facteurs peuvent causer la dégradation des composés actifs, notamment la chaleur et l'oxydation.

Le développement de compositions stabilisantes alternatives limitant la dégradation d'au moins un composé actif d'une formulation à la lumière, à la chaleur et/ou à l'oxydation, est requis.

Un des buts de la présente invention est donc de proposer une composition pour stabiliser les formulations de type précité contre les effets néfastes de la lumière, de la chaleur et/ou de l'oxydation et étant compatible avec le(s) composé(s) actif(s) présent(s) dans ces formulations.

Dans ce cadre, l'invention se fixe en particulier pour but de fournir des compositions stabilisantes utilisables en cosmétique, en dermo-cosmétique et en pharmacie.

A cet effet, l'invention a pour objet une composition comprenant :

(A) un composé A, le composé A étant le butyl-méthoxy-dibenzoylméthane ;
(B) un composé B , le composé B étant l'éthyl-hexyl-salicylate ; et
(C) un composé C, le composé C étant le benzo triazolyl dodécyl p-crésol sous forme linéaire, ramifiée ou de mélange de ces formes.

Le composé A est utile à titre de filtre ultraviolet contre les rayonnements UV-A. Le composé B est utile à titre de filtre ultraviolet contre les rayonnements UV-B.

Dans un mode de réalisation particulier, la composition de type précité comprend :

- de 5 à 90% en poids, de préférence de 22 à 35% en poids, de composé A ;
- de 5 à 90% en poids, de préférence de 22 à 35% en poids, de composé B ; et

- de 5 à 90% en poids, de préférence de 30 à 56% en poids, de composé C ;

par rapport au poids total des composés A, B et C.

L'invention a également pour objet l'utilisation de la composition de type précité pour ralentir, voire empêcher, la dégradation causée par la lumière, la chaleur et/ou l'oxydation d'au moins un composé actif choisi parmi les colorants, les molécules odorantes, les vitamines, les antioxydants et les mélanges de ceux-ci.

L'invention concerne également une formulation comprenant de 0,1 à 5% en poids de la composition de type précité.

La formulation selon l'invention peut être une formulation cosmétique, dermo-cosmétique ou pharmaceutique.

Par "filtre ultraviolet" (ou "filtre UV"), on entend, au sens de la présente description, un composé chimique capable d'absorber des rayonnements électromagnétiques dans une gamme comprise dans le domaine des rayonnements ultraviolets, à savoir dans le domaine de longueurs d'ondes comprises entre 10 et 400 nanomètres. On distingue notamment les rayonnements ultraviolets UV-A, dont la longueur d'ondes est comprise entre 315 et 400 nanomètres, les rayonnements ultraviolets UV-B, dont la longueur d'ondes est comprise entre 280 et 315 nanomètres, et les rayonnements ultraviolets UV-C, dont la longueur d'ondes est comprise entre 100 et 280 nanomètres.

L'association des composés A, B et C permet en général une absorption des rayonnements compris entre 200 et 400 nanomètres.

Les filtres UV de la famille des dibenzoylméthanes et des salicylates sont des composés bien connus qui sont décrits notamment dans la Directive C.E.E. N° 76/768 (filtres ultraviolets acceptés en cosmétique) ou dans les Directives de la F.D.A. (*Food and Drugs Administration* américaine), à savoir dans le Federal Register, vol. 43, n° 166 (25 août 1978).

Les inventeurs ont constaté que l'association des composés A, B et C assure une stabilisation très efficace d'au moins un composé actif d'une formulation à la lumière, notamment aux rayonnements ultra-violets, à la chaleur et/ou à l'oxydation. Par chaleur, on entend typiquement une température supérieure à 25°C, notamment supérieure à 30°, parfois supérieure à 35°C.

Sans vouloir être liés à une théorie particulière, les inventeurs pensent que cette stabilisation serait due au moins en partie à la limitation des phénomènes d'oxydation pouvant entraîner la dégradation du(des) composé(s) actif(s) de la formulation.

Les inventeurs ont découvert que, de façon surprenante, l'association des composés A, B et C est adaptée pour réduire la dégradation d'au moins un composé actif généralement utilisé dans les formulations cosmétiques, dermo-cosmétiques et/ou pharmaceutiques.

Le composé actif est notamment choisi parmi les colorants, les parfums, les antioxydants, comme les flavonoïdes, et les vitamines, comme le rétinol, et les mélanges de ceux-ci.

Le composé A, utile à titre de filtre UV-A, appartient à la famille des dibenzoylméthanes. Le butyl-méthoxydibenzoylméthane est aussi appelé "avobenzone".

Le composé B, utile à titre de filtre ultraviolet UV-B, appartient à la famille des salicylates.

Le composé C est un agent photostabilisant filtrant les rayonnements ultraviolets sur une large bande de longueur d'onde allant de 200 à 370 nanomètres.

Le composé C est le 2-(2H-benzotriazol-2-yl)-6-dodécyl-4-méthylphénol, aussi appelé benzo triazolyl dodécyl p-crésol, sous forme linéaire, ramifiée ou un mélange des formes linéaire et ramifiée. Ce produit est notamment commercialisé sous le nom Tinogard ® TL par la société BASF SE.

Avantageusement, le composé C n'engendre pas de cristallisation lorsqu'il est associé aux composés A et B. En outre, l'association des composés A, B et C est incolore ou présente une coloration suffisamment claire pour qu'elle ne modifie pas significativement la coloration de la formulation dans laquelle elle est ajoutée. D'autres photostabilisants ont été testés à la place du composé C, mais, associés aux composés A et B, une cristallisation et/ou une coloration, généralement jaune, ont été observés.

On considère qu'une composition comprenant un agent photostabilisant est incolore lorsque la différence d'écart de teinte dE* entre cette composition et une composition semblable exempte d'agent photostabilisant est inférieure à 4 dans l'espace colorimétrique L*a*b* CIE 1976.

La composition stabilisante est exempte de 2-éthylhexyl-méthoxycinnamate, de préférence exempte de cinnamate.

La teneur des composés A, B et C dans la composition stabilisante peut varier en une large mesure.

Toutefois, une composition stabilisante préférée comprend :

- de 5 à 90% en poids de composé A ;
- de 5 à 90% en poids de composé B ; et
- de 5 à 90% en poids de composé C

par rapport au poids total des composés A, B et C.

Cette composition est particulièrement efficace pour protéger au moins un composé actif d'une formulation contre les dégradations dues à la lumière, notamment aux rayonnements UV, à la chaleur et/ou à l'oxydation.

Sont particulièrement préférées les compositions stabilisantes qui comprennent de 10 à 40% en poids de composé A par rapport au poids total des composés A, B et C. De telles teneurs en composé A sont particulièrement avantageuse pour stabiliser un colorant. La plage de 10 à 40% est particulièrement adéquate pour protéger le colorant contre les dégradations causées par la lumière la chaleur et/ou l'oxydation. Une teneur en composé A de 28,5% par rapport à A+B+C est particulièrement avantageuse pour stabiliser le colorant.

De plus, sont particulièrement préférées les compositions stabilisantes qui comprennent au moins 10% en poids de composé B par rapport au poids total des composés A, B et C. Généralement, plus la teneur en composé B par rapport à A+B+C est élevée, plus le parfum est protégé des dégradations causées par la lumière, la chaleur et/ou l'oxydation.

Sont également particulièrement préférées les compositions stabilisantes qui comprennent au moins 10% en poids, notamment au moins 20% en poids, de préférence au moins 25% en poids, de composé C par rapport au poids total des composés A, B et C. Généralement, plus la teneur en composé C par rapport à A+B+C, est élevée, plus le colorant est protégé des dégradations causées par la lumière, la chaleur et/ou l'oxydation.

Avantageusement, la composition stabilisante comprend :

- de 10 à 40% en poids de composé A ;
- de 10 à 60% en poids de composé B ; et
- de 10 à 65% en poids de composé C

par rapport au poids total des composés A, B et C.

Selon un mode de réalisation préférentiel, la composition stabilisante comprend :

- de 22 à 35% en poids de composé A ;
- de 22 à 35% en poids de composé B ; et
- de 30 à 56% en poids de composé C

par rapport au poids total des composés A, B et C.

Ainsi, selon un mode de réalisation particulier, la composition stabilisante comprend:

- 28,5% en poids de composé A ;
- 28,5% en poids de composé B ; et
- 43% en poids de composé C

par rapport au poids total des composés A, B et C.

La composition stabilisante peut comprendre en outre au moins un solvant D.

Le solvant D est choisi parmi les solvants adaptés à une utilisation dans une formulation dans le domaine cosmétique, dermo-cosmétique et/ou pharmaceutique et dans lesquels les composés A, B et C sont solubles. Le solvant D est ainsi un solvant autorisé dans les domaines précités, notamment selon les législations européennes, américaines et/ou japonaises.

Le solvant D est avantageusement choisi parmi les solvants lipophiles, notamment parmi:

- le diméthylisosorbide ;
- le N-butylphtalimide ;
- le N-isopropylphtalimide ;
- le diméthylcapramide ;
- l'éthylhexylméthoxycrylène ;
- l'isosorbide dicaprylate ;
- le phényl éthyl benzoate ;
- le dipropylène glycol dibenzoate ;
- l'isopropyl lauryl sarcosinate ;
- le PPG-15 stéaryl éther benzoate ;
- le diisopropyl adipate ;
- l'éthylhexylbenzoate ;
- le phénoxycaprylate ;
- le diéthylhexyl maléate ;
- le C12-15 alkyl benzoate ;

- le diéthylhexyl 2,6-naphtalate ;
- le butyloctyl salicylate ;

et un mélange de ceux-ci.

Dans un mode de réalisation préférentiel, le solvant D est choisi parmi :

- le N-butylphthalimide ;
- le N-isopropylphthalimide ;
- le C12-15 alkyl benzoate ;
- le dipropylène glycol dibenzoate ;
- le PPG-15 stéaryl éther ;

et un mélange de ceux-ci.

Par exemple, le solvant D est un mélange de C12-15 alkyl benzoate, de dipropylène glycol dibenzoate et de PPG-15 stéaryl éther.

Le composé D est avantageusement un mélange de N-butylphthalimide et de N-isopropylphthalimide.

Lorsque la composition stabilisante comporte un solvant D, le solvant D représente généralement de 10% à 40%, de préférence du poids total de la composition stabilisante, de préférence 30% du poids total de la composition stabilisante.

Le composé A est soluble dans la plupart des solvants organiques, notamment dans les alcools et dans les phases lipidiques. Les composés B, C et le solvant D sont miscibles avec la plupart des solvants organiques, notamment les phases lipidiques. Le composé B et le solvant D sont aussi miscibles avec les alcools. De ce fait, la composition stabilisante, comprenant un solvant D ou exempt de solvant D, est aisément incorporée dans les formulations cosmétiques, dermo-cosmétiques et/ou pharmaceutiques comportant des solvants organiques ou des tensioactifs telles que les shampooings, les bains moussants, les eaux de toilette, eaux de parfums et huiles.

La composition stabilisante peut se présenter sous différentes formes en fonction de la destination envisagée.

La composition stabilisante se présente avantageusement sous forme liquide à 20°C, de préférence à une température supérieure ou égale à 15°C et inférieure ou égale à 35°C, à pression atmosphérique (1 bar).

Aussi, la composition stabilisante se présente sous forme liquide à température ambiante, ce qui en facilite le dosage et permet une incorporation efficace et homogène dans une formulation cosmétique

La composition stabilisante peut comprendre en outre un tensioactif. Le tensioactif facilite la solubilisation de la composition stabilisante dans des formulations à base de solvants hydrophiles, et notamment dans des formulations aqueuses.

Des tensioactifs particulièrement adaptés sont notamment les tensioactifs non ioniques et en particulier le mélange de tensioactifs non ioniques commercialisé par la société LCW sous le nom de solubilisant LRI.

Le tensioactif est mis en oeuvre dans une quantité suffisante pour assurer la dispersion de la composition stabilisante dans la formulation. Généralement, une quantité de 60 à 80%, et de préférence de l'ordre de 70% en poids par rapport au poids total des composés A, B et C est appropriée.

Selon cette variante, la composition stabilisante ajoutée à la formulation forme une émulsion ou micro-émulsion, dans laquelle la phase huileuse comprend les composés A, B et C.

Les compositions stabilisantes décrites permettent de limiter la dégradation par la lumière, la chaleur et/ou l'oxydation d'au moins un composé actif d'une formulation comprenant une phase organique et/ou une phase aqueuse. Ainsi, la formulation stabilisée peut être de type solution, émulsion eau dans l'huile ou huile dans l'eau, ou gel.

La présente invention a également pour objet un procédé de fabrication de la composition stabilisante précitée, comprenant le mélange des composés A, B et C. Lorsque la composition comprend un solvant D, le procédé de fabrication de la composition comporte typiquement les étapes suivantes :

a) Préparation d'une solution du composé A dans le solvant D, de préférence en chauffant un mélange du composé A et du solvant D, à 40°C,
b) Ajout du composé B à la solution obtenue à l'étape a), typiquement à 40°C pour obtenir un mélange,
c) Ajout du composé C au mélange obtenu à l'étape b), typiquement à 40°C pour obtenir un mélange,
d) Agitation du mélange obtenu à l'étape c) jusqu'à homogénéisation, ce par quoi la composition stabilisante est obtenue. Ladite composition est alors apte à être ajoutée à une formulation cosmétique, dermo-cosmétique et/ ou pharmaceutique.

En variante, le procédé de fabrication de la composition comporte typiquement les étapes suivantes :

a') Préparation d'une solution des composés B et C dans le solvant D, de préférence en agitant et en chauffant à 40°C un mélange du composé B, du composé C et du solvant D,

b') Ajout du composé A à la solution obtenue à l'étape a'), typiquement à 40°C pour obtenir un mélange,

c') Agitation du mélange obtenu à l'étape b') jusqu'à homogénéisation, ce par quoi la composition stabilisante est obtenue. Ladite composition est alors apte à être ajoutée à une formulation cosmétique, dermo-cosmétique et/ ou pharmaceutique.

L'invention a également pour objet l'utilisation de la composition précitée pour ralentir, voire empêcher, la dégradation causée par la lumière, la chaleur et/ou l'oxydation d'au moins un composé actif choisi parmi les colorants, les molécules odorantes, les vitamines, les antioxydants et les mélanges de ceux-ci. L'invention concerne également l'utilisation de la composition définie ci-dessus pour protéger au moins un tel composé actif contre une dégradation causée par la lumière, la chaleur et/ou l'oxydation. L'invention concerne un procédé pour ralentir, voire empêcher, la dégradation causée par la lumière, la chaleur et/ou l'oxydation d'au moins un composé actif comprenant la mise en contact d'un composé actif choisi parmi les colorants, les molécules odorantes, les vitamines, les antioxydants et les mélanges de ceux-ci (ou avec une formulation, notamment cosmétique, comprenant un tel composé actif) avec une composition telle que définie ci-dessus.

Le rétinol est un exemple de vitamine. Les flavonoïdes sont des exemples d'antioxydants.

Par exemple, les colorants sont choisis parmi le FD&C Yellow 5, le FD&C Red 4, le D&C Red 33, le Ext D&C Violet 2, le FD&C Blue 1 et les mélanges de ceux-ci.

De préférence, la composition est utilisée pour ralentir, voire empêcher, la dégradation causée par la lumière, la chaleur et/ou l'oxydation d'au moins un colorant, d'au moins une molécule odorante et/ou d'un mélange de ceux-ci.

L'invention concerne également l'utilisation de la composition comme antioxydant.

A cette fin, la composition définie ci-dessus est ajoutée à une formulation cosmétique, telle qu'un parfum, une eau de toilette, un produit de maquillage, comme un fond de teint, ou encore un produit de soin ou d'hygiène corporelle tels qu'un shampooing ou un gel douche.

On ajoute la composition stabilisante à la formulation en une quantité appropriée qui dépend de la nature du composé actif, mais aussi du degré d'exposition de la formulation aux rayons UV et à la chaleur. Elle est donc notamment fonction de la transparence du récipient contenant la formulation aux rayons UV et de la durée de vie attendue pour la formulation.

En principe, une teneur de 0,1 à 5%, de préférence de 0,2% à 2%, et tout particulièrement de 0,3% à 1,0% en poids de composition stabilisante par rapport au poids total de la formulation est suffisante.

Dans le cas particulier de composition aqueuse, il est en général préférable que le mélange des composés A, B et C soit mis en oeuvre à raison de 1,5 à 4% en poids par rapport au poids total de la formulation.

Selon un autre aspect, la présente invention a pour objet une formulation, notamment cosmétique, dermo-cosmétique ou pharmaceutique, comprenant la composition telle que définie précédemment.

La formulation stabilisée peut être de type solution, émulsion, ou gel.

La formulation est de préférence un parfum, une eau de toilette, un produit de maquillage, comme un fond de teint, ou encore un produit de soin ou d'hygiène corporelle tel qu'un shampooing ou un gel douche

La formulation peut comprendre une phase huileuse telle que, par exemple, une huile polaire, une huile apolaire ou un parfum, ou bien encore une phase hydro-alcoolique, comprenant au moins 10%, voire au moins 30% en poids d'un alcool tel que l'éthanol.

Si la formulation comprend une phase aqueuse ou hydro-alcoolique, la composition stabilisante peut être solubilisée grâce à un tensioactif. Le tensioactif peut être déjà présent dans la formulation, et/ou dans la composition stabilisante ajoutée.

L'invention concerne également l'utilisation de la composition définie ci-dessus pour ralentir, voire empêcher, la dégradation causée par la lumière, la chaleur et/ou l'oxydation d'une formulation cosmétique.

D'autres avantages et caractéristiques de la présente invention apparaîtront au vu des exemples illustratifs exposés ci-après.

## EXEMPLE 1: Préparation de compositions stabilisantes

Dix compositions stabilisantes comprenant du butyl-méthoxy-dibenzoylméthane (composé A), de l'éthyl-hexyl-salicylate (composé B) et du benzo triazolyl dodécyl p-crésol (composé C) dans du Pelemol® BIP PC (solvant D) ont été préparées suivant un protocole comportant les étapes suivantes :

a) Préparation d'une solution de butyl-méthoxy-dibenzoylméthane dans du Pelemol® BIP PC en chauffant à 40°C au bain-marie,

b) Ajout de l'éthyl-hexyl-salicylate à la solution obtenue à l'étape a) pour obtenir un mélange,

c) Ajout du benzo triazolyl dodécyl p-crésol au mélange obtenu à l'étape b), à 40°C, pour obtenir un mélange,

d) Agitation du mélange obtenu à l'étape c) jusqu'à homogénéisation, ce par quoi la composition stabilisante est obtenue.

Le Pelemol® BIP PC est un mélange eutectique de N-butylphthalimide et de N-isopropylphthalimide commercialisé par la société Phoenix Chemical, Inc..

Les compositions stabilisantes ont été préparées suivant les compositions indiquées dans le tableau 1. Les pourcentages sont exprimés par rapport au poids total de la composition.

Tableau 1 : Compositions stabilisantes

|  | 1 | 2 | 3 | 4 | 5 | 6 | 7 | 8 | 9 | 10 |
|---|---|---|---|---|---|---|---|---|---|---|
| Composé A | 20,0% | 27,0% | 20,0% | 20,0% | 27,0% | 27,0% | 23,5% | 20,0% | 23,5% | 27,0% |
| Composé B | 20,0% | 20,0% | 5,0% | 12,5% | 20,0% | 12,5% | 5,0% | 20,0% | 20,0% | 5,0% |
| Composé C | 30,0% | 10,0% | 30,0% | 30,0% | 20,0% | 10,0% | 10,0% | 10,0% | 30,0% | 20,0% |
| Composé D | qsp. 100% | qsp. 100% | qsp. 100% | qsp. 100% | qsp. 100% | qsp. 100% | qsp. 100% | qsp. 100% | qsp. 100% | qsp. 100% |

Les compositions stabilisantes 1 à 10 ont été ajoutées à différentes formulations comprenant chacune un mélange colorant/parfum selon les indications du tableau 2.

Les pourcentages sont exprimés par rapport au poids total de la formulation.

Tableau 2 : Formulations comprenant les compositions stabilisantes

| Formulation | Parfum | Colorant (solution à 0,1%) | Composition stabilisante | |
|---|---|---|---|---|
| F1 | 546202209 Magical Amber | FD&C Red 4 | 2 | 1,00% |
| F2 | 546202209 Magical Amber | FD&C Red 4 | 3 | 1,00% |
| F3 | 546202209 Magical Amber | Ext D&C Violet 2 | 4 | 1,00% |
| F4 | 546202426 Mineral Splash | FD&C Red 4 | 5 | 1,00% |
| F5 | 546202209 Magical Amber | FD&C Yellow 5 | 3 | 0,30% |
| F6 | 546202209 Magical Amber | FD&C Yellow 5 | 6 | 0,30% |
| F7 | 546202209 Magical Amber | FD&C Yellow 5 | 7 | 1,00% |
| F8 | 546202209 Magical Amber | FD&C Yellow 5 | 8 | 0,65% |
| F9 | 546202258 Diamond Beauty | FD&C Yellow 5 | 8 | 1,00% |
| F10 | 546202258 Diamond Beauty | FD&C Yellow 5 | 9 | 0,30% |
| F11 | 546202258 Diamond Beauty | FD&C Yellow 5 | 10 | 1,00% |
| F12 | 546202258 Diamond Beauty | FD&C Yellow 5 | 3 | 0,65% |
| F13 | 546202425 Oriental Infinity | FD&C Yellow 5 | 1 | 1,00% |

Chaque formulation comprenait :

- 8% en poids de parfum ;
- 1% en poids d'une solution comprenant 0,1% en poids de colorant dans de l'eau déminéralisée ;
- de 0,3% à 1% en poids de composition stabilisante ;
- 75% en poids d'éthanol ; et
- de l'eau déminéralisée.

Les pourcentages sont exprimés par rapport au poids total de la formulation.

Les parfums sont commercialisés par la société Sensient Fragrances et les colorants sont commercialisés par la société Sensient Cosmetic Technologies.

A titre de comparaison, on a réalisé des formulations F1' à F13' similaires respectivement aux formulations F1 à F13, dans lesquelles on a remplacé les compositions stabilisantes 1 à 13 par une composition comparative. Cette composition, ci-après dénommée « Composition comparative », consiste en :

- 15% en poids d'éthylhexyl salicylate ;
- 15% en poids d'avobenzone ; et
- 70% en poids de 2-éthylhexyl-méthoxycinnamate.

Les différentes formulations réalisées ont été soumises à différents tests afin d'évaluer les performances des compositions stabilisantes.

Chaque formulation a été divisée en quatre échantillons :

- un est placé dans une étuve à 45°C pendant trente jours (échantillon 1) ;
- un est placé au réfrigérateur à 4°C durant un mois (échantillon 2) ;
- un est exposé pendant six semaines à la lumière naturelle (LN) (échantillon 3) ; et
- un est exposé à la lumière artificielle (S24) dans un appareil SUN-TEST de chez Heraeus pendant 24 heures (échantillon 4) ;

*Test de résistance à la chaleur (test 1)*

L'échantillon 1 et l'échantillon 2 sont évalués olfactivement suivant le protocole suivant.

Deux testeurs comparent olfactivement les échantillons 1 et 2.

Si les deux testeurs considèrent que les échantillons 1 et 2 présentent la même odeur, l'échantillon 1 reçoit la note « + ».

Si les deux testeurs considèrent que les échantillons 1 et 2 ne présentent pas la même odeur, l'échantillon 1 reçoit la note « - ».

Si les deux testeurs sont en désaccord, un juge expert compare olfactivement les échantillons 1 et 2. Si le juge expert considère que les échantillons 1 et 2 présentent la même odeur, l'échantillon 1 reçoit la note « + ». Sinon, l'échantillon 1 reçoit la note « - ».

*Test de résistance à la lumière naturelle (LN) (test 2)*

L'échantillon 3 est caractérisé dans l'espace colorimétrique L*a*b* CIE 1976, aussi appelé CIELAB 1976, en utilisant l'étalon colorimétrique D65.

L'écart total de teinte dE* dans l'espace colorimétrique CIELAB 1976 entre l'échantillon 3 et un témoin ayant la même composition que l'échantillon testé, sauf qu'il est exempt de composition stabilisante, le témoin ayant été conservé pendant six semaines dans l'obscurité est ensuite calculé. dE* (LN) correspond à la distance géométrique entre le témoin et l'échantillon 3 dans ledit espace.

Plus dE* est élevé, plus l'échantillon 3 a subi une modification de sa coloration sous l'effet de la lumière naturelle.

*Test SUNTEST (test 3)*

L'échantillon 4 est caractérisé dans l'espace colorimétrique L*a*b* CIE 1976, aussi appelé CIELAB 1976, en utilisant l'étalon colorimétrique D65.

L'écart total de teinte dE* dans l'espace colorimétrique CIELAB 1976 est ensuite calculé entre l'échantillon 4 et un témoin ayant la même composition que l'échantillon testé, sauf qu'il est exempt de composition stabilisante, le témoin ayant été conservé dans l'obscurité. dE*(S24) correspond à la distance géométrique entre le témoin et l'échantillon 4 dans ledit espace.

Plus dE* est élevé, plus l'échantillon 4 a subi une modification de sa coloration sous l'effet de la lumière naturelle.

Stabilisation à la chaleur

L'effet stabilisant à la chaleur des compositions stabilisantes a été évalué suivant le test 1 décrit précédemment.

Les résultats du test 1 sont exposés dans le tableau 3 ci-dessous.

Tableau 3 : Stabilisation à la chaleur des formulations comprenant les compositions stabilisantes

| Formulation | F1 | F4 | F5 | F6 | F8 | F9 | F10 | F12 | F13 |
|---|---|---|---|---|---|---|---|---|---|
| Stabilisation à la chaleur | + | + | + | + | + | + | + | + | + |

Ces résultats montrent un net effet de stabilisation à la chaleur.

A titre comparatif, l'effet stabilisant à la chaleur de la Composition comparative (introduite à la même concentration dans la formulation) a été évalué suivant le même test de mesure olfactif décrit précédemment.

Les résultats de ce test sont exposés dans le tableau 4 ci-dessous.

Tableau 4 : Stabilisation à la chaleur des formulations comprenant la Composition comparative

| Formulation comparative | F1' | F4' | F5' | F6' | F8' | F9' | F10' | F12' | F13' |
|---|---|---|---|---|---|---|---|---|---|
| Stabilisation à la chaleur | + | + | - | - | - | - | - | - | + |

Stabilisation à la lumière

L'effet stabilisant à la lumière des compositions stabilisantes a été évalué suivant les tests 2 et 3 décrits précédemment.

Les résultats des deux tests sont exposés dans le tableau 5 ci-après.

Tableau 5 : Stabilisation à la lumière (naturelle (LN) et artificielle (S24)) de formulations comprenant les compositions stabilisantes par rapport à des témoins exempts de composition stabilisante et conservés à l'obscurité

| | F1 | F2 | F3 | F4 | F5 | F6 | F7 | F8 | F9 | F10 | F11 | F12 | F13 |
|---|---|---|---|---|---|---|---|---|---|---|---|---|---|
| dE* (LN) | 19,9 | 6,7 | 1,1 | 3 | 31,1 | 31,9 | 6,1 | 17 | 10,3 | 7,9 | 0,4 | 1,9 | 0,7 |
| dE* (S24) | 24 | 16,7 | 10,6 | 4,7 | 36,1 | 36,1 | 31,7 | 35,3 | 4,3 | 19,6 | 0,8 | 7 | 3,2 |

il n'y a pas de valeur limite de dE* acceptable, car, d'une couleur à l'autre, la variation de la couleur est plus ou moins perceptible à l'œil. Par exemple, l'œil humain est plus sensible aux variations de couleur sur des teintes rouges plutôt que sur des teintes jaunes. Cela étant, l'objectif est d'obtenir une valeur dE* la plus faible possible, car plus dE* est faible, plus la composition stabilisante a stabilisé à la lumière la formulation qui la contient. La composition stabilisante 1 a particulièrement stabilisé la formulation F13 qui la contient. La composition stabilisante 10 a particulièrement stabilisé la formulation F11 qui la contient.

**EXEMPLE 2: Etude d'une composition stabilisante préférentielle** : **Stabilisation à la lumière artificielle (Sun-Test S24)**

On a réalisé une composition stabilisante, dite « Composition préférentielle », comprenant 28,5% en poids de butyl-méthoxy-benzoyl-méthane (composé A), 28,5% en poids d'éthyl-hexyl-salicylate (composé B) et 43% en poids de benzo triazolyl dodecyl p-cresol (composé C) par rapport au poids de A+B+C selon le protocole décrit ci-après.

Dans un mélangeur, on a introduit 28,5 g de composé A dans 43 g de Pelemol® BIP PC (solvant D). Le mélange a été chauffé à 40°C au bain-marie jusqu'à obtenir la dissolution complète du composé A.

Puis, 28,5 g de composé B a été ajouté à la solution du composé A dans le solvant D, à 40°C.

Ensuite, 43 g de composé C a été introduit dans le mélangeur et l'ensemble a été chauffé à 40°C au bain-marie sous agitation jusqu'à obtention d'une composition homogène.

La composition préférentielle contient donc 70% de matière active (A+B+C) et 30 de solvant D.

**Formulations comprenant la composition stabilisante**

La Composition préférentielle a été ajoutée à différentes formulations comprenant chacune un colorant et un parfum.

1% de composition préférentielle ont été ajoutés dans les différentes formulations, soit 0,7% de matière active (A+B+C).

a - **Colorant FD&C Red 4**

Les formulations a1 à a8 ont été réalisées selon les indications du tableau 6. Les pourcentages sont exprimés par rapport au poids total de la formulation.

Tableau 6 : Formulations comprenant le colorant FD&C Red 4

| Formulations | a1 | a2 | a3 | a4 | a5* | a6* | a7* | a8* |
|---|---|---|---|---|---|---|---|---|
| FD&C Red 4 (solution à 0,1%) | 1% | 1% | 1% | 1% | 1% | 1% | 1% | 1% |
| 546202209 Magical Amber | 8% | - | - | - | 8% | - | - | - |
| 546202425 Oriental Infinity | - | 8% | - | - | - | 8% | - | - |
| 546202258 Diamond Beauty | - | - | 8% | - | - | - | 8% | - |
| 546202426 Mineral Splash | - | - | - | 8% | - | - | - | 8% |
| Composition préférentielle | 1% | 1% | 1% | 1% | - | - | - | - |
| éthanol | 75% | 75% | 75% | 75% | 75% | 75% | 75% | 75% |
| eau déminéralisée | q.s. 100% | q.s. 100% | q.s. 100% | q.s. 100% | q.s. 100% | q.s. 100% | q.s. 100% | q.s. 100% |
| * témoin exempt de composition stabilisante | | | | | | | | |

b - **Colorant D&C Red 33**

Les formulations b1 à b8 ont été réalisées selon les indications du tableau 7. Les pourcentages sont exprimés par rapport au poids total de la formulation.

Tableau 7 : Formulations comprenant le colorant D&C Red 33

| Formulations | b1 | b2 | b3 | b4 | b5* | b6* | b7* | b8* |
|---|---|---|---|---|---|---|---|---|
| D&C Red 33 (solution à 0,1%) | 1% | 1% | 1% | 1% | 1% | 1% | 1% | 1% |
| 546202209 Magical Amber | 8% | - | - | - | 8% | - | - | - |
| 546202425 Oriental Infinity | - | 8% | - | - | - | 8% | - | - |
| 546202258 Diamond Beauty | - | - | 8% | - | - | - | 8% | - |
| 546202426 Mineral Splash | - | - | - | 8% | - | - | - | 8% |
| Composition préférentielle | 1% | 1% | 1% | 1% | - | - | - | - |
| éthanol | 75% | 75% | 75% | 75% | 75% | 75% | 75% | 75% |
| eau déminéralisée | q.s. 100% | q.s. 100% | q.s. 100% | q.s. 100% | q.s. 100% | q.s. 100% | q.s. 100% | q.s. 100% |
| * témoin exempt de composition stabilisante | | | | | | | | |

## c - Colorant FD&C Yellow 5

Les formulations c1 à c8 ont été réalisées selon les indications du tableau 8. Les pourcentages sont exprimés par rapport au poids total de la formulation.

Tableau 8 : Formulations comprenant le colorant FD&C Yellow 5

| Formulations | c1 | c2 | c3 | c4 | c5* | c6* | c7* | c8* |
|---|---|---|---|---|---|---|---|---|
| FD&C Yellow 5 (solution à 0,1 %) | 1% | 1% | 1% | 1% | 1% | 1% | 1% | 1% |
| 546202209 Magical Amber | 8% | - | - | - | 8% | - | - | - |
| 546202425 Oriental Infinity | - | 8% | - | - | - | 8% | - | - |
| 546202258 Diamond Beauty | - | - | 8% | - | - | - | 8% | - |
| 546202426 Mineral Splash | - | - | - | 8% | - | - | - | 8% |
| Composition préférentielle | 1% | 1% | 1% | 1% | - | - | - | - |
| éthanol | 75% | 75% | 75% | 75% | 75% | 75% | 75% | 75% |
| eau déminéralisée | q.s. 100% | q.s. 100% | q.s. 100% | q.s. 100% | q.s. 100% | q.s. 100% | q.s. 100% | q.s. 100% |

* témoin exempt de composition stabilisante

## d - Colorant Ext D&C Violet 2

Les formulations d1 à d8 ont été réalisées selon les indications du tableau 9. Les pourcentages sont exprimés par rapport au poids total de la formulation.

Tableau 9 : Formulations comprenant le colorant Ext D&C Violet 2

| Formulations | d1 | d2 | d3 | d4 | d5* | d6* | d7* | d8* |
|---|---|---|---|---|---|---|---|---|
| Ext D&C Violet 2 (solution à 0,1%) | 1% | 1% | 1% | 1% | 1% | 1% | 1% | 1% |
| 546202209 Magical Amber | 8% | - | - | - | 8% | - | - | - |
| 546202425 Oriental Infinity | - | 8% | - | - | - | 8% | - | - |
| 546202258 Diamond Beauty | - | - | 8% | - | - | - | 8% | - |
| 546202426 Mineral Splash | - | - | - | 8% | - | - | - | 8% |
| Composition préférentielle | 1% | 1% | 1% | 1% | - | - | - | - |
| éthanol | 75% | 75% | 75% | 75% | 75% | 75% | 75% | 75% |

11

(suite)

| Formulations | d1 | d2 | d3 | d4 | d5* | d6* | d7* | d8* |
|---|---|---|---|---|---|---|---|---|
| eau déminéralisée | q.s. 100% | q.s. 100% | q.s. 100% | q.s. 100% | q.s. 100% | q.s. 100% | q.s. 100% | q.s. 100% |
| * témoin exempt de composition stabilisante | | | | | | | | |

Les formulations a1 à a8, b1 à b8, c1 à c8 et d1 à d8 ont été exposées à des rayonnements ultraviolets pendant 24 heures dans un appareil SUN TEST, suivant le test 3. A l'issue de ce traitement, on a mesuré l'écart de teinte dE* comme défini ci-dessus.

Le taux d'efficacité est calculé en comparant l'écart de teinte dE* d'une formulation comprenant la Composition préférentielle (a1 à a4, b1 à b4, c1 à c4 et d1 à d4) et celui d'un mélange témoin (a5 à a8, b5 à b8, c5 à c8 et d5 à d8) comportant le même colorant et le même parfum et dans lequel aucune composition stabilisante n'a été introduite suivant la formule suivante :

$$taux\ d'efficacité = \frac{(dE^*_{témoin} - dE^*_{formulation})}{dE^*_{témoin}}$$

Le taux d'efficacité permet donc de comparer par rapport à une formulation exempte de composition stabilisante.

Tableau 10 : Formulations comprenant le colorant FD&C Red 4

| Compositions | a1 | a2 | a3 | a4 | a5* | a6* | a7* | a8* |
|---|---|---|---|---|---|---|---|---|
| dE* | 16,04 | 13,09 | 5,47 | 22,45 | 35,88 | 30,76 | 30,78 | 30,71 |
| Taux d'efficacité | 55,3 | 57,4 | 82,2 | 26,9 | - | - | - | - |
| * témoin exempt de composition stabilisante | | | | | | | | |

Tableau 11 : Formulations comprenant le colorant D&C Red 33

| Compositions | b1 | b2 | b3 | b4 | b5* | b6* | b7* | b8* |
|---|---|---|---|---|---|---|---|---|
| dE* | 22,3 | 20,03 | 12,77 | 20,22 | 35,66 | 48,25 | 48,24 | 48,12 |
| Taux d'efficacité | 37,5 | 58,5 | 73,5 | 58,0 | - | - | - | - |
| * témoin exempt de composition stabilisante | | | | | | | | |

Tableau 12 : Formulations comprenant le colorant FD&C Yellow 5

| Compositions | c1 | c2 | c3 | c4 | c5* | c6* | c7* | c8* |
|---|---|---|---|---|---|---|---|---|
| dE* | 21,47 | 0,53 | 1,73 | 30,39 | 34,89 | 34,89 | 36,08 | 35,66 |
| Taux d'efficacité | 38,5 | 98,5 | 95,2 | 14,8 | - | - | - | - |
| * témoin exempt de composition stabilisante | | | | | | | | |

Tableau 13 : Formulations comprenant le colorant Ext D&C Violet 2

| Compositions | d1 | d2 | d3 | d4 | d5* | d6* | d7* | d8* |
|---|---|---|---|---|---|---|---|---|
| dE* | 12,18 | 11,37 | 8,34 | 12,99 | 20,38 | 23,84 | 23,56 | 24,15 |
| Taux d'efficacité | 40,2 | 52,3 | 64,6 | 46,2 | - | - | - | - |
| * témoin exempt de composition stabilisante | | | | | | | | |

Ces résultats montrent clairement un très net effet de stabilisation de la coloration obtenu lorsque l'on introduit la Composition préférentielle dans les différentes formulations.

**EXEMPLE 3 comparatif** : Stabilisation à la lumière artificielle (Sun-Test S24)

A titre comparatif, on a réalisé des formulations similaires à celles de l'exemple 2, dans lesquelles la Composition préférentielle a été remplacée par la « Composition comparative » de l'exemple 1. La « Composition comparative », consiste en :

- 15% en poids d'éthylhexyl salicylate ;
- 15% en poids d'avobenzone ; et
- 70% en poids de 2-éthylhexyl-méthoxycinnamate

et est donc exempte de solvant.

Les formulations ont été testées dans les mêmes conditions que dans l'exemple 2 (exposition aux rayonnements ultraviolets pendant 24 heures).

Le taux d'efficacité est calculé en comparant l'écart de teinte dE* d'une formulation comprenant la Composition comparative et celui d'un mélange témoin comportant le même colorant et le même parfum et dans lequel aucune composition stabilisante n'a été introduite suivant la formule suivante :

$$taux\ d'efficacité = \frac{(dE^*_{témoin} - dE^*_{formulation})}{dE^*_{témoin}}$$

Le taux d'efficacité permet donc de comparer par rapport à une formulation exempte de composition stabilisante.

Les résultats observés sont exposés dans les tableaux 14 à 17 ci-après.

Pour évaluer quelle composition stabilisante est la plus efficace entre la composition préférentielle et la composition comparative pour chaque mélange colorant/parfum, les taux d'efficacité sont comparés entre eux et la différence $\Delta$ est calculée suivant la formule suivante :

$$\Delta = (taux\ d'efficacité_{composition\ préférentielle} - taux\ d'efficacité_{composition\ comparative})$$

Si la différence $\Delta$ est supérieure ou égale à 5%, on considère que la Composition préférentielle de l'exemple 2 permet une meilleure stabilisation de la formulation à la lumière que la Composition comparative.

Si la différence $\Delta$ est comprise de -5% à 5%, on considère que la Composition préférentielle de l'exemple 2 et la Composition comparative présentent la même efficacité en termes de stabilisation à la lumière.

Si la différence $\Delta$ est inférieure ou égale à -5%, on considère que la Composition comparative permet une meilleure stabilisation de la formulation à la lumière que la Composition préférentielle de l'exemple 2.

Tableau 14 : Formulations comprenant le colorant FD&C Red 4

| Formulations | a9 | a10 | a11 | a12 |
|---|---|---|---|---|
| FD&C Red 4 (solution à 0,1%) | 1% | 1% | 1% | 1% |
| 546202209 Magical Amber | 8% | - | - | - |
| 546202425 Oriental Infinity | - | 8% | - | - |
| 546202258 Diamond Beauty | - | - | 8% | - |
| 546202426 Mineral Splash | - | - | - | 8% |
| Composition comparative | 0,3% | 0,3% | 0,3% | 0,3% |
| éthanol | 75% | 75% | 75% | 75% |
| eau déminéralisée | q.s. 100% | q.s. 100% | q.s. 100% | q.s. 1 00% |
| dE* | 34,84 | 21,63 | 4,79 | 26,05 |
| Taux d'efficacité | 2,9% | 29,7% | 84,4% | 15,2% |

(suite)

| Formulations | a9 | a10 | a11 | a12 |
|---|---|---|---|---|
| Δ | 52,4% | 27,8% | - 2,2% | 11,7% |

Tableau 15 : Formulations comprenant le colorant D&C Red 33

| Formulations | b9 | b10 | b11 | b12 |
|---|---|---|---|---|
| D&C Red 33 (solution à 0,1%) | 1% | 1% | 1% | 1% |
| 546202209 Magical Amber | 8% | - | - | - |
| 546202425 Oriental Infinity | - | 8% | - | - |
| 546202258 Diamond Beauty | - | - | 8% | - |
| 546202426 Mineral Splash | - | - | - | 8% |
| Composition comparative | 0,3% | 0,3% | 0,3% | 0,3% |
| éthanol | 75% | 75% | 75% | 75% |
| eau déminéralisée | q.s. 100% | q.s. 100% | q.s. 100% | q.s. 1 00% |
| dE* | 30,88 | 19,24 | 5,01 | 12,35 |
| Taux d'efficacité | 13,4% | 60,1% | 89,6% | 74,3% |
| Δ | 24,4% | -1,6% | -16,1% | -16,3% |

Tableau 16 : Formulations comprenant le colorant FD&C Yellow 5

| Formulations | c9 | c10 | c11 | c12 |
|---|---|---|---|---|
| FD&C Yellow 5 (solution à 0,1%) | 1% | 1% | 1% | 1% |
| 546202209 Magical Amber | 8% | - | - | - |
| 546202425 Oriental Infinity | - | 8% | - | - |
| 546202258 Diamond Beauty | - | - | 8% | - |
| 546202426 Mineral Splash | - | - | - | 8% |
| Composition comparative | 0,3% | 0,3% | 0,3% | 0,3% |
| éthanol | 75% | 75% | 75% | 75% |
| eau déminéralisée | q.s. 100% | q.s. 100% | q.s. 100% | q.s. 1 00% |
| dE* | 33,29 | 20,22 | 3,91 | 33,84 |
| Taux d'efficacité | 4,6% | 42,0% | 89,2% | 5,1% |
| Δ | 33,9% | 56,4% | 6,0% | 9,7% |

Tableau 17 : Formulations comprenant le colorant Ext D&C Violet 2

| Formulations | d9 | d10 | d11 | d12 |
|---|---|---|---|---|
| Ext D&C Violet 2 (solution à 0,1%) | 1% | 1% | 1% | 1% |
| 546202209 Magical Amber | 8% | - | - | - |
| 546202425 Oriental Infinity | - | 8% | - | - |
| 546202258 Diamond Beauty | - | - | 8% | - |
| 546202426 Mineral Splash | - | - | - | 8% |

(suite)

| Formulations | d9 | d10 | d11 | d12 |
|---|---|---|---|---|
| Composition comparative | 0,3% | 0,3% | 0,3% | 0,3% |
| éthanol | 75% | 75% | 75% | 75% |
| eau déminéralisée | q.s. 100% | q.s. 100% | q.s. 100% | q.s. 100% |
| dE* | 18,73 | 9,01 | 7,83 | 13,2 |
| Taux d'efficacité | 8,1% | 62,2% | 66,8% | 45,3% |
| Δ | 32,1% | -9,9% | -2,2% | 0,9% |

Il ressort des tableaux 14 à 17 que la Composition préférentielle de l'exemple 2 présente la même efficacité ou une meilleure efficacité en termes de stabilisation à la lumière que la Composition comparative dans la majorité des formulations étudiées.

**EXEMPLE 4: Etude d'une composition stabilisante préférentielle** : **Stabilisation à la chaleur**

L'effet stabilisant à la chaleur de la Composition préférentielle a été évalué.

Un échantillon de chaque formulation a1 à a8, b1 à b8, c1 à c8 et d1 à d8 a été conservé dans l'obscurité à 4°C pendant 30 jours et un autre échantillon de chaque formulation a1 à a8, b1 à b8, c1 à c8 et d1 à d8 a été conservé dans l'obscurité à 45°C pendant 30 jours.

Les échantillons ont ensuite été caractérisés dans l'espace colorimétrique L*a*b* CIE 1976, aussi appelé CIELAB 1976, en utilisant l'étalon colorimétrique D65.

L'écart total de teinte dE* dans l'espace colorimétrique CIELAB 1976 entre chaque échantillon et un témoin conservé à 4°C est ensuite calculé. dE* correspond à la distance géométrique entre le témoin et l'échantillon dans ledit espace.

La perte de teinte dE*$_{/4°C}$ a été calculée en comparant la teinte de l'échantillon conservé à 4°C à celle de l'échantillon conservé à 45°C suivant la formule suivante, dans l'espace colorimétrique CIELAB 1976 :

$$dE^*_{/4°C} = \sqrt{(L_{45°C} - L_{4°C})^2 + (a_{45°C} - a_{4°C})^2 + (b_{45°C} - b_{4°C})^2}$$

Plus dE*$_{/4°C}$ est faible, meilleure est la stabilisation de la coloration à la chaleur. Les résultats de ces tests sont exposés dans les tableaux 18 à 21 ci-dessous.

Tableau 18 : Formulations comprenant le colorant FD&C Red 4

| Compositions | a1 | a2 | a3 | a4 | a5* | a6* | a7* | a8* |
|---|---|---|---|---|---|---|---|---|
| dE*$_{/4°C}$ | 2,60 | 1,36 | 0,86 | 1,56 | 5,05 | 2,02 | 2,71 | 1,09 |
| * témoin exempt de composition stabilisante | | | | | | | | |

Tableau 19 : Formulations comprenant le colorant D&C Red 33

| Compositions | b1 | b2 | b3 | b4 | b5* | b6* | b7* | b8* |
|---|---|---|---|---|---|---|---|---|
| dE*$_{/4°C}$ | 9,33 | 1,78 | 1,85 | 0,45 | 9,06 | 4,63 | 5,43 | 2,68 |
| * témoin exempt de composition stabilisante | | | | | | | | |

Tableau 20 : Formulations comprenant le colorant FD&C Yellow 5

| Compositions | c1 | c2 | c3 | c4 | c5* | c6* | c7* | c8* |
|---|---|---|---|---|---|---|---|---|

(suite)

| Compositions | c1 | c2 | c3 | c4 | c5* | c6* | c7* | c8* |
|---|---|---|---|---|---|---|---|---|
| dE*$_{/4°C}$ | 1,42 | 0,93 | 0,88 | 2,30 | 1,38 | 1,94 | 1,94 | 3,08 |
| * témoin exempt de composition stabilisante | | | | | | | | |

Tableau 21 : Formulations comprenant le colorant Ext D&C Violet 2

| Compositions | d1 | d2 | d3 | d4 | d5 | d6 | d7 | d8 |
|---|---|---|---|---|---|---|---|---|
| dE*$_{/4°C}$ | 2,70 | 1,26 | 0,79 | 0,39 | 6,20 | 2,85 | 3,27 | 1,53 |

Dans chaque tableau, il faut comparer des compositions ayant le même colorant et le même parfum, soit pour chaque tableau, respectivement :

- les formulations 1 (avec composition préférentielle) et 5 (exempte de composition stabilisante),
- les formulations 2 (avec composition préférentielle) et 6 (exempte de composition stabilisante),
- les formulations 3 (avec composition préférentielle) et 7 (exempte de composition stabilisante),
- les formulations 4 (avec composition préférentielle) et 8 (exempte de composition stabilisante).

Ces résultats montrent clairement une nette diminution de la dégradation de la coloration à la chaleur lorsque l'on introduit la Composition préférentielle dans les différentes formulations.

**EXEMPLE 5 comparatif** : **Stabilisation à la chaleur**

A titre comparatif, les compositions a9 à a12, b9 à b12, c9 à c12 et d9 à d12 ont été testées dans les mêmes conditions que dans l'exemple 4.

Les résultats observés sont exposés dans les tableaux 22 à 25 ci-après.

Tableau 22 : Formulations comprenant le colorant FD&C Red 4

| Formulations | a9 | a10 | a11 | a12 |
|---|---|---|---|---|
| dE*$_{/4°C}$ | 4,41 | 1,36 | 1,03 | 0,64 |

Tableau 23 : Formulations comprenant le colorant D&C Red 33

| Formulations | b9 | b10 | b11 | b12 |
|---|---|---|---|---|
| dE*$_{/4°C}$ | 9,69 | 2,01 | 2,67 | 0,48 |

Tableau 24 : Formulations comprenant le colorant FD&C Yellow 5

| Formulations | c9 | c10 | c11 | c12 |
|---|---|---|---|---|
| dE*$_{/4°C}$ | 2,15 | 1,05 | 0,52 | 0,36 |

Tableau 25 : Formulations comprenant le colorant Ext D&C Violet 2

| Formulations | d9 | d10 | d11 | d12 |
|---|---|---|---|---|
| dE*$_{/4°C}$ | 5,09 | 1,52 | 1,20 | 0,50 |

Il faut comparer des compositions ayant le même colorant et le même parfum, et donc respectivement les dE*$_{/4°C}$ des formulations suivantes :

- 1 (composition préférentielle) et 9 (composition comparative),
- 2 (composition préférentielle) et 10 (composition comparative),
- 3 (composition préférentielle) et 11 (composition comparative),
- 4 (composition préférentielle) et 12 (composition comparative).

Il ressort de la comparaison des tableaux 18 à 21 (composition préférentielle) et des tableaux 22 à 25 (composition comparative) que la Composition préférentielle de l'exemple 2 présente la même efficacité ou une meilleure efficacité en termes de stabilisation de la coloration à la chaleur que la Composition comparative dans la majorité des formulations étudiées.

**EXEMPLE 6 : Pouvoir antioxydant**

6.1. de la Composition préférentielle

Le pouvoir antioxydant de la Composition préférentielle de l'exemple 2 et de ses composés a été mesuré grâce à la technologie PAOT Liquid Technology ® développée par l'Institut Européen des Antioxydants (IEA). Il est exprimé avec le score PAOT ® qui mesure la capacité d'un produit à neutraliser des radicaux libres.
Les résultats sont exprimés dans le tableau 26 ci -dessous.

Tableau 26 : Pouvoir antioxydant

|  | Composé A | Composé B | Composé C | Solvant D | Composition préférentielle |
|---|---|---|---|---|---|
| Pouvoir antioxydant (score PAOT ®) | 0,17/g | 164,33/L | 172,50/L | 165,83/L | 158,00/L |

La composition préférentielle présente un score PAOT ® de 158/L, avec une déviation standard de 0,50.
Le pouvoir antioxydant d'un litre de Composition préférentielle et de chacun de ses composés a été comparé au pouvoir antioxydant de différents antioxydants de référence. Les résultats de cette comparaison sont exprimés en poids d'antioxydant de référence équivalent dans le tableau 27 ci-dessous.

Tableau 27 : Comparaison des pouvoirs antioxydants

|  | Vitamine E | Vitamine C | Trolox | BHA | BHT |
|---|---|---|---|---|---|
| Composé A (/g) | 0,04 | $1,48.10^{-2}$ | $2,16.10^{-2}$ | $1,67.10^{-2}$ | $1,90.10^{-2}$ |
| Composé B (/L) | 35,39 | 14,1 | 20,6 | 16,0 | 18,1 |
| Composé C (/L) | 37,15 | 14,8 | 21,6 | 16,7 | 19,0 |
| Solvant D (/L) | 35,71 | 14,3 | 20,8 | 16,1 | 18,3 |
| Composition préférentielle (/L) | 34,03 | 13,6 | 19,8 | 15,3 | 17,4 |

Le pouvoir antioxydant (PAOT Score ®) d'un litre de Composition préférentielle équivaut ainsi au pouvoir antioxydant de 34,03 g de vitamine E, de 13,6 g de vitamine C, de 19,8 g de trolox, de 15,3 g d'hydroxyanisole butylé (BHA) ou de 17,4 g d'hydroxytoluène butylé (BHT).

6.2. de la composition comparative et d'une composition ne comprenant que les composes A et B.

Une composition ne comprenant que les composés A et B comme matière active a été préparée en mélangeant 20% en poids de A, 20% en poids de B et 60% en poids de solvant D (Pelemol® BIP PC).
Les pouvoirs oxydants de cette composition, de la composition comparative et de la composition préférentielle ont été mesurés. Les résultats des équivalences antioxydantes (en g/L de produit) sont fournis au tableau 28 :

Tableau 28 : Comparaison des pouvoirs antioxydants

|  | Vitamine C | BHA | BHT | $\alpha$-tocopherol |
|---|---|---|---|---|
| Composition comparative (/L) | 4,81 | 5,43 | 6,16 | 12,05 |
| Mélange composés A et B (/L) | 6,54 | 7,38 | 8,37 | 16,37 |

(suite)

| | Vitamine C | BHA | BHT | α-tocopherol |
|---|---|---|---|---|
| Composition préférentielle (/L) | 13,6 | 15,3 | 17,4 | 34,03 |

Les résultats du tableau 28 montrent un pouvoir antioxydant plus élevé avec la composition préférentielle qu'avec la composition comparative ou le mélange des composés A et B.

**EXEMPLE 7** : **Comparaison de la stabilisation à la lumière (sun-test) de la composition préférentielle ou de la composition comparative, lorsqu'elles sont utilisées avec la même proportion de matière active**

Trois séries de tests sont réalisées afin de comparer l'effet stabilisant à la lumière de la composition préférentielle et de la composition comparative lorsqu'elles sont utilisées avec la même proportion de matières actives, à savoir avec 0,68%, 1% ou 2% de matières actives.

Comme détaillé à l'exemple 2, la composition préférentielle contient 70% de matière active (A+B+C) et 30% de solvant D. Comme détaillé à l'exemple 3, la composition stabilisante contient 100% de matière active (pas de solvant).

Chaque composition stabilisante a été introduite dans une formulation comprenant, en exprimant les pourcentages sont exprimés par rapport au poids total de la formulation

- 8% en poids de parfum Oriental Infinity ;
- 1% en poids d'une solution comprenant 0,1% en poids de colorant FD&C Yellow 5 dans de l'eau déminéralisée ;
- une concentration en poids de composition stabilisante telle que précisée au tableau 29 ;
- 75% en poids d'éthanol ; et
- le restant (qsp 100%) en poids d'eau déminéralisée.

L'écart total de teinte dE* dans l'espace colorimétrique CIELAB 1976 est ensuite calculé entre la formulation obtenue et un témoin ayant la même composition que la formulation testée, sauf qu'il est exempt de composition stabilisante, le témoin ayant été conservé dans l'obscurité.

Tableau 29 : Comparaison de la stabilisation obtenue par la composition préférentielle ou comparative, lorsqu'elles sont utilisées avec la même concentration en matière active dans la formulation testée

| Composition stabilisante | Concentration en composition stabilisante | Concentration en matière active | dE* |
|---|---|---|---|
| préférentielle | 0,97% | 0,68% | 11 |
| comparative | 0,68% | 0,68% | 11 |
| préférentielle | 1,43% | 1% | 5 |
| comparative | 1,% | 1% | 7 |
| préférentielle | 2,43% | 2% | 1 |
| comparative | 2% | 2% | 2 |

La stabilisation à la lumière est donc identique ou améliorée avec la composition stabilisante préférentielle selon l'invention par rapport à la composition stabilisante comparative.

**EXEMPLE 8** : **Influence de la présence et de la concentration en composé C sur la stabilisation à la lumière**

Des compositions stabilisantes différant par la teneur en composé C sont préparées. Leurs compositions sont détaillées au tableau 30.

Tableau 30 : Compositions des compositions stabilisantes différant par la teneur en composé C

| Composition | C0% (comparative) | C10% | C20% | C30% |
|---|---|---|---|---|
| Composé A (%wt) | 20 | 20 | 20 | 20 |
| Composé B (%wt) | 20 | 20 | 20 | 20 |

(suite)

| Composition | C0% (comparative) | C10% | C20% | C30% |
|---|---|---|---|---|
| Composé C (%wt) | 0 | 10 | 20 | 30 |
| Solvant D (%wt) | 60 | 50 | 40 | 30 |

Ces compositions stabilisantes ont chacune été ajoutées à une formulation selon les indications du tableau 31. Les pourcentages sont exprimés par rapport au poids total de la formulation.

Tableau 31 : Formulations comprenant les compositions stabilisantes

| Formulation | Parfum | Colorant (solution à 0,1%) | Composition stabilisante | |
|---|---|---|---|---|
| FC0% | Oriental Infinity | FD&C Yellow 5 | C0% | 1,00% |
| FC10% | Oriental Infinity | FD&C Yellow 5 | C10% | 1,00% |
| FC20% | Oriental Infinity | FD&C Yellow 5 | C20% | 1,00% |
| FC30% | Oriental Infinity | FD&C Yellow 5 | C30% | 1,00% |

Chaque formulation comprenait :

- 8% en poids de parfum Oriental Infinity ;
- 1% en poids d'une solution comprenant 0,1% en poids de colorant FD&C Yellow 5 dans de l'eau déminéralisée ;
- 1% en poids de composition stabilisante ;
- 75% en poids d'éthanol ; et
- 15% en poids d'eau déminéralisée.

Les pourcentages sont exprimés par rapport au poids total de la formulation.

Les quatre formulations réalisées ont été exposées à la lumière artificielle dans un appareil SUN-TEST de chez Heraeus pendant 24 heures, puis caractérisées dans l'espace colorimétrique L*a*b* CIE 1976 en utilisant l'étalon colorimétrique D65. L'écart total de teinte dE* a été calculé entre chaque formulation ayant subi le sun-test pendant t = 24h et un témoin ayant exactement la même composition que la formulation testée (y compris la composition stabilisante) mais à t=0 (juste après sa préparation). Plus dE* est élevé, plus la formulation a subi une modification de sa coloration sous l'effet de la lumière. Les résultats sont fournis au tableau 32.

Tableau 32 : Stabilisation à la lumière artificielle (sun-test S24) de formulations comprenant des compositions stabilisantes à différentes proportions du composé C par rapport à des témoins exempts de composition stabilisante et conservés à l'obscurité

| | FC0% | FC10% | FC20% | FC30% |
|---|---|---|---|---|
| dE* (S24) | 20,07 | 10,22 | 1,62 | 0,81 |

La présence de C dans la composition stabilisante permet donc de stabiliser la formulation la comprenant. La stabilisation est d'autant plus effective que la concentration en C est élevée dans la composition stabilisante.

## Revendications

1.  Composition comprenant :

    (A) de 5 à 90% en poids d'un composé A, le composé A étant le butyl-méthoxy-dibenzoylméthane ;
    (B) de 5 à 90% en poids d'un composé B, le composé B étant l'éthyl-hexyl-salicylate ; et
    (C) de 5 à 90% en poids d'un composé C, le composé C étant le benzo triazolyl dodécyl p-crésol sous forme linéaire, ramifiée ou un mélange des formes linéaire et ramifiée,
    les proportions en composés A, B et C étant par rapport au poids total des composés A, B et C, et
    la composition étant exempte de 2-éthylhexyl-méthoxycinnamate.

**2.** Composition selon la revendication 1, comprenant :

- de 10 à 40% en poids, de préférence de 22 à 35% en poids, de composé A ;
- de 10 à 60% en poids, de préférence de 22 à 35% en poids, de composé B ; et
- de 10 à 65% en poids, de préférence de 30 à 56% en poids, de composé C

par rapport au poids total des composés A, B et C.

**3.** Composition selon l'une quelconque des revendications précédentes, qui se présente sous forme liquide à une température supérieure ou égale à 15°C et inférieure ou égale à 35°C, à pression atmosphérique.

**4.** Composition selon l'une quelconque des revendications précédentes, comprenant un solvant D.

**5.** Composition selon la revendication 4, dans laquelle le solvant D est choisi parmi :

- le N-butylphthalimide ;
- le N-isopropylphthalimide ;
- le C12-15 alkyl benzoate ;
- le dipropylène glycol dibenzoate ;
- le PPG-15 stéaryl éther,

et un mélange de ceux-ci, le solvant D étant de préférence un mélange de N-butylphthalimide et de N-isopropylphthalimide.

**6.** Composition selon la revendication 4 ou 5, dans laquelle le solvant D représente de 10% à 40% du poids total de la composition.

**7.** Utilisation de la composition selon l'une quelconque des revendications 1 à 6 pour ralentir, voire empêcher, la dégradation causée par la lumière et/ou la chaleur et/ou l'oxydation d'au moins un composé actif choisi parmi les colorants, les molécules odorantes, les vitamines, les antioxydants et les mélanges de ceux-ci.

**8.** Formulation comprenant de 0,1 à 5% en poids de la composition selon l'une des revendications 1 à 6.

**9.** Formulation selon la revendication 8, comprenant un colorant choisi parmi le FD&C Red 4, le Ext D&C Violet 2, le FD&C Blue 1 et les mélanges de ceux-ci.

**10.** Formulation selon la revendication 8 ou 9, qui est une formulation cosmétique, dermo-cosmétique ou pharmaceutique.

**Patentansprüche**

**1.** Zusammensetzung, umfassend:

(A) von 5 bis 90 Gewichts-% einer Verbindung A, wobei die Verbindung A Butylmethoxydibenzoylmethan ist;
(B) von 5 bis 90 Gewichts-% einer Verbindung B, wobei die Verbindung B Ethylhexylsalicylat ist; und
(C) 5 bis 90 Gewichts-% einer Verbindung C, wobei die Verbindung C Benzotriazolyldodecyl-p-cresol in linearer, verzweigter Form oder ein Gemisch der linearen und verzweigten Form ist,
wobei die Anteile an den Verbindungen A, B und C auf das Gesamtgewicht der Verbindungen A, B und C bezogen sind, und
wobei die Zusammensetzung frei von 2-Ethylhexylmethoxycinnamat ist.

**2.** Zusammensetzung nach Anspruch 1, umfassend:

- von 10 bis 40 Gewichts-%, vorzugsweise von 22 bis 35 Gewichts-%, von Verbindung A;
- von 10 bis 60 Gewichts-%, vorzugsweise von 22 bis 35 Gewichts-%, von Verbindung B; und
- von 10 bis 65 Gewichts-%, vorzugsweise von 30 bis 56 Gewichts-%, von Verbindung C

bezogen auf das Gesamtgewicht der Verbindungen A, B und C.

3. Zusammensetzung nach einem der vorherigen Ansprüche, die bei einer Temperatur von 15 °C oder höher und 35 °C oder niedriger bei atmosphärischem Druck in flüssiger Form vorliegt.

4. Zusammensetzung nach einem der vorherigen Ansprüche, umfassend ein Lösungsmittel D.

5. Zusammensetzung nach Anspruch 4, wobei das Lösungsmittel D ausgewählt ist aus:

   - N-Butylphthalimid;
   - N-Isopropylphthalimid;
   - C12-15-Alkylbenzoat;
   - Dipropylenglycoldibenzoat;
   - PPG-15-Stearylether,

   und einem Gemisch davon, wobei das Lösungsmittel D vorzugsweise ein Gemisch aus N-Butylphthalimid und N-Isopropylphthalimid ist.

6. Zusammensetzung nach Anspruch 4 oder 5, wobei das Lösungsmittel D 10 % bis 40 % des Gesamtgewichts der Zusammensetzung darstellt.

7. Verwendung der Zusammensetzung nach einem der Ansprüche 1 bis 6, um den durch Licht und/oder Hitze und/oder Oxidation verursachten Abbau von mindestens einer aktiven Verbindung, die ausgewählt ist aus Farbstoffen, Duftmolekülen, Vitaminen, Antioxidantien und Gemischen, zu verlangsamen oder sogar zu verhindern.

8. Formulierung, umfassend 0,1 bis 5 Gewichts-% der Zusammensetzung nach einem der Ansprüche 1 bis 6.

9. Formulierung nach Anspruch 8, umfassend einen Farbstoff, der ausgewählt ist aus FD&C Red 4, Ext D&C Violet 2, FD&C Blue 1 und Gemischen davon.

10. Formulierung nach Anspruch 8 oder 9, die eine kosmetische, dermokosmetische oder pharmazeutische Formulierung ist.

**Claims**

1. A composition comprising:

   (A) from 5 to 90 weight % of compound A, compound A being butyl-methoxydibenzoylmethane;
   (B) from 5 to 90 weight % of compound B, compound B being ethyl-hexyl salicylate; and
   (C) from 5 to 90 weight % of compound C, compound C being benzo triazolyl dodecyl p-cresol in linear or branched form, or a mixture of linear and branched forms,
   the proportions of compounds A, B and C being relative to the total weight of compounds A, B and C, and
   the composition being free of 2-ethylhexyl-methoxycinnamate.

2. The composition according to claim 1, comprising

   - from 10 to 40 weight %, preferably from 22 to 35 weight % of compound A;
   - from 10 to 60 weight %, preferably from 22 to 35 weight % of compound B; and
   - from 10 to 65 weight %, preferably from 30 to 56 weight % of compound C, relative to the total weight of compounds A, B and C.

3. The composition according to any of the preceding claims that is in liquid form at a temperature higher than or equal to 15 °C and lower than or equal to 35 °C, at atmospheric pressure.

4. The composition according to any of the preceding claims comprising a solvent D.

5. The composition according to claim 4, wherein the solvent D is chosen from among:

- N-butylphthalimide;
- N-isopropylphthalimide;
- C12-15 alkyl benzoate;
- dipropylene glycol dibenzoate;
- PPG-15 Stearyl ether,

and a mixture thereof, solvent D preferably being a mixture of N-butylphthalimide and N-isopropylphthalimide.

6. The composition according to claim 4 or 5, wherein solvent D represents from 10 % to 40 % of the total weight of the composition.

7. Use of the composition according to any of claims 1 to 6 to slow, even prevent degradation caused by light and/or heat and/or oxidation of at least one active compound chosen from among colourants, odorant molecules, vitamins, antioxidants and mixtures thereof.

8. A formulation comprising from 0.1 to 5 weight % of the composition according to one of claims 1 to 6.

9. The formulation according to claim 8 comprising a colourant chosen from among FD&C Red 4, Ext D&C Violet 2, FD&C Blue 1 and mixtures thereof.

10. The formulation according to claim 8 or 9 that is a cosmetic, dermo-cosmetic or pharmaceutical formulation.

## RÉFÉRENCES CITÉES DANS LA DESCRIPTION

**Documents brevets cités dans la description**

- EP 1755543 B1

**Littérature non-brevet citée dans la description**

- *Federal Register,* 25 Août 1978, vol. 43 (166
- Filtres et écrans solaires. **M.C. POELMAN.** Actifs et additifs en cosmétologie. Editions Tec & Doc, 1999
- *Federal Register,* 25 Août 1978, vol. 3